# EUROPEAN PATENT APPLICATION

(11) **EP 3 964 191 A1**
(43) Date of publication of application: **09.03.2022**
(21) Application number: 21194370.9
(22) Date of filing: 01.09.2021
(51) Int. Cl.: A61K 8/29, A61K 31/192, A61K 31/7004, A61K 36/185, A61K 36/45, A61K 36/738, A61P 17/00, A61K 36/484, A61K 8/365, A61K 8/60, A61K 8/9711, A61K 8/9789, A61Q 19/02, A61Q 19/08

(54) **FORMULATION FOR THE TREATMENT OF SKIN DYSCHROMIA**

(30) Priority: 08.09.2020 IT 202000021238
(71) Applicant: NOVA S.r.l., Roma (IT)
(72) Inventor: Tagliaferri, Vincenzo, Roma (IT); Statti, Giancarlo, Roma (IT)
(74) Representative: Cioncoloni, Giuliana

(57) **Abstract**

This invention relates to a formulation for the treatment of skin dyschromia which, thanks to its active ingredients, inhibits tyrosinase, promotes cell turnover, and acts as a UVA and UVB filter.

## Description

### FIELD OF THE INVENTION

This invention refers to the field of pharmacology, cosmetology, and dermatology, and relates to a formulation for the treatment of skin dyschromia.

### STATE OF THE ART

Skin disorders like melasma, hyperpigmentation, acne, eczema, and dermatitis are very common skin dysfunctions. The world's elderly population is growing, and exposure to various pollutants, the use and abuse of chemicals and drugs, hormonal changes, unhealthy nutritional habits, exposure to the sun, and their prolonged effects over time, cause the formation of skin blemishes and disorders. Of these, one of the most common is the formation of skin dyschromia or hyperpigmentation.

Skin disorders like hyperpigmentation and other pigmentation conditions of irregular skin are undesirable and unsightly. For example, the occurrence of acne, rashes, scratches, or injuries to the skin may cause a post-inflammatory hyperpigmentation characterized by the presence of unwanted dark spots on the face or on other parts of the body. Hyperpigmentation is characterized by the darkening of an area of the skin, caused by the overproduction of pigment in the skin known as melanin. Hyperpigmentation refers to the areas of the skin where excess melanin has been produced and deposited, causing spots on the skin that appear darker than the surrounding skin.

Hyperpigmentation of the skin is caused by a superabundant concentration of melanocytes that produce melanin, or by hyperactive melanocytes. For example, exposure to the sun stimulates melanin production. Although hyperpigmentation can strike anyone, it is more common among certain ethnic groups, such as Asian, Mediterranean, African, or Latino. Hyperpigmentation can strike any part of the body, including face, hands, and neck.

Many products are available for skin care, for the treatment of skin disorders and blemishes like acne and spots. The chemical ingredients in many of these products are irritants or are even harmful to the skin. Alternative compounds are also available to lighten skin color.

However, most of the synthetic compounds in use have side effects, and it has been reported that they provide only temporary effects, with hyperpigmentation reappearing if the use of this compound is interrupted.

Therefore, the need exists for a formulation for the treatment of skin dyschromia that brings desirable results for overcoming the aforementioned troubles, and that has no side effects.

### DESCRIPTION OF THE INVENTION

The primary purpose of this invention is to provide a formulation for the treatment of skin dyschromia that allows the formation of spots on the skin to be prevented and reduced.

Another purpose of this invention is to obtain an anti-aging action thanks to its antioxidant-rich composition.

A further purpose of this invention is to provide a formulation for the treatment of skin dyschromia that is stable, non-toxic, and without side effects of any kind.

Yet another purpose of this invention is to provide a formulation for the treatment of skin dyschromia that can be applied on a daily basis for a prolonged period of time with no side effects.

An additional purpose of this invention is to provide a formulation for the treatment of skin dyschromia in which the active ingredients are evenly distributed.

Lastly, a purpose of this invention is to provide a formulation for the treatment of skin dyschromia that is soothing and has a characteristic pleasant odor.

This invention will be fully included based on the following description provided merely by way of example and not of limitation.

This invention, then, proposes attaining the purposes discussed above by developing a formulation for the treatment of skin dyschromia which, according to claim 1, includes:
- Titanium dioxide between 1% and 3% by weight;
- Mandelic acid between 0.05% and 0.2% by weight;
- Arbutin between 0.05% and 0.3% by weight;
- Glycolic extract of bearberry leaves between 2% and 5% by weight;
- Glycerine extract of pomegranate between 1% and 3% by weight;
- Glycerine extract of rose hips between 0.5% and 2% by weight;
- Glycerine extract of licorice between 0.5% and 2% by weight;
- Sea oak extract between 1% and 3% by weight;
- At least one pharmaceutically acceptable excipient.

As a benefit, the formulation for the treatment of skin dyschromia that is the object of this invention includes several natural extracts obtained from the plant world, which, thanks to their properties, have an anti-aging action and allow skin hyperpigmentation to be prevented and reduced without incurring side effects due to prolonged use.

As a benefit, the formulation for the treatment of skin dyschromia that is the object of this invention, thanks to its active ingredients, inhibits tyrosinase, promotes cellular turnover, and acts as a UVA and UVB filter.

The formulation that is the object of this invention has a pleasant fragrance, permits regular, frictionless application, has evenly distributed active ingredients, is stable, and can be easily applied.

The main ingredient of glycolic extract of bearberry leaves is arbutin, which is currently the most effective depigmentation agent that acts by selectively blocking the tyrosinase, thus inhibiting the production of melanin.

Moreover, its preventive action guarantees a sure effect as a skin dyschromia antagonist.

The extract of pomegranate is marked by the high content of ellagic acid and anthocyanin, which gives it significant antioxidant properties; it is also depigmenting. The pomegranate acts upon pigmentation and inhibits melanogenesis, which is to say the formation of new melanin giving rise to spots.

The rose hip is a plant abundant in ascorbic acid (vitamin C), flavonoids, and above all anthocyanidins, which perform an intense antioxidant activity. This antioxidant activity combats the oxidation phenomena underlying the melanogenesis, and accelerates skin cell turnover.

One of the most important active ingredients present in the licorice is glabrin, which inhibits tyrosinase activity. Liquitin is highly important, since it seems to lighten the skin by dispersing the melanin. The glycerine extract of licorice is the darker pigment, functioning as depigmentation agent with a smaller number of side effects.

The brown algae Halidrys siliquosa or sea oak is known for being rich in phloroglucinol oligomers capable of inhibiting the melanogenesis chain reaction by focusing on the various passages that take place before, during, and after melanin synthesis. Sea oak extract can block the overproduction of melanin and consequently diminish skin pigmentation, acting as a lightener starting as early as the first 10 days of application. The plant parts used in this invention may be derived from barks, roots, tubers, stigma, pits, exudates, stolons, rhizomes, leaves, seeds, nuts, berries, fruits, stems, and flowers.

Depending on the forms of development of this invention, an effective dose formulation for the treatment of skin dyschromia, between 0.5 grams and 1 gram, is given once a day, preferably in the evening.

Preferably, this invention is ivory, cream or white in color.

This invention's formulation for the treatment of skin dyschromia has effect of lightening / depigmenting the skin, the ability to reduce melanocytes, and no side effects. Therefore, the formulation for the treatment of skin dyschromia is safe and can be effectively used to alleviate symptoms of hyperpigmentation, melasma, chloasma, and various skin pigmentation disorders.

The term "an effective quantity" refers to the quantity of this invention's formulation that is required to confer one of the effects described above, like reducing the signs of pigmentation, reducing dark spots, reducing dark post-acne spots, and having an anti- aging effect.

The formulation for the treatment of skin dyschromia that is the object of this invention has a quick, long-lasting effect, is non-toxic, and has no side effects. It also has good stability and is suited for mass production.

The ingredients of the composition also possess antibacterial, antimycotic, anti-inflammatory, and antimicrobial properties, and therefore help give relief to patients with hyperpigmentation, melasma, and other skin disorders.

This invention's formulation can therefore be in the form of a preparation facilitating topical application, like a cream, gel, emulsion, and liquid.

This invention has been described and illustrated with reference to specific forms of its development, but it must be expressly understood that variations, additions, and/or omissions may be made without bringing it, on these grounds, out of the sphere of protection, which remains defined only in the attached claims.

## Claims

1. Formulation for the treatment of skin hyperpigmentation which, according to claim 1, includes:
- Titanium dioxide between 1% and 3% by weight;
- Mandelic acid between 0.05% and 0.2% by weight;
- Arbutin between 0.05% and 0.3% by weight;
- Glycolic extract of bearberry leaves between 2% and 5% by weight;
- Glycerine extract of pomegranate between 1% and 3% by weight;
- Glycerine extract of rose hips between 0.5% and 2% by weight;
- Glycerine extract of licorice between 0.5% and 2% by weight;
- Sea oak extract between 1% and 3% by weight;
- At least one pharmaceutically acceptable.

2. Formulation as claimed in claim 1 which can be in cream, gel, emulsion and liquid form.

3. Formulation as claimed in the preceding claims which can be ivory, cream or white in color.

4. Formulation as claimed in the preceding claims for the treatment of skin dyschromia.

5. Formulation as claimed in the preceding claims for the treatment of skin dyschromia which also has an anti-aging effect.

6. Formulation as claimed in the previous claims in which the effective daily dose is between 0.5 grams and 1 gram and is given in a single application, preferably in the evening.
